# EUROPÄISCHE PATENTSCHRIFT

(11) **EP 3 199 095 B1**
(45) Veröffentlichungstag und Bekanntmachung des Hinweises auf die Patenterteilung: **26.12.2018**
(21) Anmeldenummer: 17155795.2
(22) Anmeldetag: 22.09.2015
(51) Int. Cl.: A61B 3/00, A61B 3/103

(54) **SYSTEME ZUR BESTIMMUNG DER AUGENREFRAKTION**
SYSTEM FOR THE DETERMINATION OF EYE REFRACTION
SYSTEME POUR LA DETERMINATION DE LA REFRACTION OCULAIRE

(30) Priorität: 22.09.2014 DE 102014113680; 14.11.2014 DE 102014116665
(43) Veröffentlichungstag der Anmeldung: 02.08.2017
(62) Teilanmeldung aus: 15777628.7
(73) Patentinhaber: Carl Zeiss AG, 73447 Oberkochen (DE); Carl Zeiss Vision International GmbH, 73430 Aalen (DE)
(72) Erfinder: LINDIG, Karsten, 99084 Erfurt (DE); CABEZA-GUILLÉN, Jesús-Miguel, 73434 Aalen (DE)
(74) Vertreter: Sticht, Andreas

(56) Entgegenhaltungen:
- WO-A2-2013/036629
- US-A1- 2004 032 568
- US-A1- 2009 153 796
- US-A1- 2011 157 550
- US-A1- 2013 027 668
- US-A1- 2013 083 185
- US-A1- 2014 211 167

## Beschreibung

Die vorliegende Anmeldung betrifft Systeme zum Bestimmen der Augenrefraktion und entsprechende Computerprogrammprodukte. Unter einer Augenrefraktion ist dabei eine Bestimmung einer Refraktion eines oder beider Augen eines Lebewesens, insbesondere eines Menschen zu verstehen. Dabei werden üblicherweise Werte für Sphäre (in Dioptrien), Zylinder und Achse bestimmt. Eine derartige Bestimmung der Augenrefraktion wird beispielsweise benutzt, um Sehhilfen wie Brillen oder Kontaktlinsen an eine jeweilige Person anzupassen.

Im medizinischen Bereich sind herkömmliche Diagnosesysteme zur Bestimmung der Augenrefraktion in der Regel als einzelne Table-Top-Systeme ausgeführt. Zur objektiven Bestimmung der Augenrefraktion wird dabei beispielsweise ein Autorefraktor oder ein Oszilloskop verwendet. Neben der objektiven Refraktionsbestimmung mittels derartiger Geräte, bei denen eine objektive Messung der Refraktion des Auges erfolgt, werden häufig auch sogenannte subjektive Refraktionsbestimmungen durchgeführt, bei welchem einer Person verschiedene Linsen oder andere optische Elemente mit verschiedenen optischen Eigenschaften bereitgestellt werden (beispielsweise in einem Brillengestell mit einer Halterung für derartige Linsen) und die Person angibt, ob sich ein Seheindruck verbessert oder verschlechtert. Hierfür betrachtet die Person üblicherweise eine Sehtafel mit Zeichen oder Symbolen, welche sich in größerer Entfernung, beispielsweise 4 m, befindet.

Somit wird herkömmlicherweise für die Bestimmung der Augenrefraktion relativ viel Platz benötigt. Zudem sind häufig hierfür nötige Vorrichtungen relativ teuer. Daher ist es wünschenswert, kompaktere Vorrichtungen zur Bestimmung der Augenrefraktion bereitzustellen.

Aus der US 2013/0235346 A1 ist es bekannt, ein Smartphone oder andere Computereinrichtungen zur Augenuntersuchung, insbesondere zur Photorefraktion, zu verwenden. Dabei wird mittels einer Kamera des Smartphones ein Auge oder werden beide Augen einer zu untersuchenden Person aufgenommen, und eine Beleuchtung wird beispielsweise über einen Blitz oder andere Leuchteinrichtung des Smartphones bereitgestellt.

Aus der US 2012/0212598 A1 ist es bekannt, ein zu untersuchendes Auge mittels einer Leuchtdiodenanordnung zu beleuchten, um ein Auge aus verschiedenen Richtungen beleuchten zu können. Es wird hier also wiederum ein spezialisiertes Gerät bereitgestellt, welches unter Umständen vergleichsweise teuer ist.

Aus der DE 10153397 A1 sind weitere Verfahren und Vorrichtungen zur Messung der Refraktion eines Auges bekannt, bei welchen ein Auge beleuchtet wird und dann ein Bild des Auges ausgewertet wird.

Die US 2013/0027668 A1 offenbart ein Verfahren zur subjektiven Refraktionsbestimmung, wofür eine mobile Anzeigevorrichtung, insbesondere ein Smartphone, verwendet werden kann. Dabei werden Bilder angezeigt, die von einem Probanden zur Deckung gebracht werden müssen.

Aus der DE 10 2008 012 268 A1 ist eine stationäre Vorrichtung zur objektiven Refraktionsbestimmung bekannt.

Die US 2006/0110008A1 offenbart Verfahren und Vorrichtungen zur kalibrierungsfreien Augennachverfolgung ("eye gaze tracking"),

Die DE 20 2011 103 183 U1 offenbart einen Bildtrenner, welcher zur Bestimmung von Defiziten der Sehschärfe und Refraktion eines Auges an einem Tablet-Rechner angebracht werden kann.

Die US 2009/0153796 A1 offenbart eine Vorrichtung mit einer am Kopf anzubringenden Einheit und einer zentralen Recheneinheit. Die Recheneinheit steuert die am Kopf getragene Einheit an, Testmuster darzustellen, um eine Refraktion von Linsen an einen Benutzer anzupassen.

Die WO 2013/170091 A1 offenbart Verfahren und Vorrichtungen zur Messung der visuellen Empfindlichkeit, wobei die Bestimmung unter verschiedenen Beleuchtungsbedingungen erfolgen kann.

Die US 2011/0157550 A1 offenbart, Leuchtdiodenmuster zu erzeugen, welche zur Bestimmung von Retinaeigenschaften oder Refraktionseigenschaften dienen.

Die US 2013/0027668 A1 offenbart eine Vorrichtung zur subjektiven Refraktionsbestimmung, bei welcher eine Helligkeit einstellbar ist, um ein Hintergrundbild zu beleuchten.

Die US 2013/0083185 A1 offenbart ein optisches Adaptersystem für eine ophthalmologische bildgebende Vorrichtung.

Die DE 103 48 854 A1 offenbart ein Verfahren und eine Vorrichtung zur Ermittlung der Restfehlsichtigkeit eines Patienten, bei welcher ein Strahlteiler zwischen einem Auge einer zu untersuchenden Person und einer Sehtafel angeordnet ist, wobei der Strahlteiler Licht von dem Auge zu einem Kamerachip lenkt. Polarisatoren dienen dabei zur Infrarotbeleuchtung und zur Unterdrückung von Linsenreflexionen.

Die US 2012/0274902 A1 offenbart eine tabletförmige Messeinrichtung zur Augenvermessung, welche mit einem Computer kommuniziert. Zur Beleuchtung können verschiedene Leuchtdioden aktiviert werden, sodass ein Pupillenreflex entsprechend wandert.

Die DE 432 760 A1 offenbart eine optische Anordnung zum Betrachten eines Displays, welche zur Prüfung zentraler Sehfunktionen verwendet werden kann.

Die US 2007/0200927 A1 offenbart es, die Sehschärfe mittels einer am Kopf zu tragenden Vorrichtung als Funktion der Helligkeit zu messen.

Ausgehend hiervon ist es eine Aufgabe der vorliegenden Erfindung, Systeme zur Bestimmung der Augenrefraktion bereitzustellen, welche beispielsweise verglichen mit herkömmlicheren Herangehensweisen kostengünstiger zu implementieren sind und/oder eine erhöhte Genauigkeit bieten und/oder flexibler einsetzbar sind als herkömmliche stationäre Einrichtungen.

Es wird ein System nach Anspruch 1 bereitgestellt. Die Unteransprüche definieren weitere Ausführungsbeispiele.

Erfindungsgemäß wird ein System bereitgestellt, umfassend:
eine am Kopf tragbare Halterung,
eine in der Halterung bereitgestellte mobile Computervorrichtung (10; 20; 60), umfassend:
   eine Anzeige (11; 21; 61),
   einen Prozessor (13), und
   einen Speicher mit darin gespeichertem Programmcode (16),
wobei der Programmcode (16), wenn er auf dem Prozessor (13) ausgeführt wird, bewirkt, dass der Prozessor (13) die Anzeige (11) ansteuert, um auf der Anzeige (11) ein Bild zur Refraktionsbestimmung eines Auges (610, 611) anzuzeigen,
wobei in der Halterung bereitgestellt sind:
   eine Betrachtungsoptik (62, 63, 100, 101) zum Betrachten der mobilen Computervorrichtung mit einem linken Auge (610) und einem rechten Auge (611) einer zu untersuchenden Person, umfassend Optiken (62, 63), um das Bild auf der Anzeige zu dem linken Auge (610) bzw. dem rechten Auge (611) abzubilden, und eine Wechselhalterung (143) für eine Wechseloptik (140, 141) zur subjektiven Refraktionsbestimmung.

Hiermit ist eine subjektive Refraktionsbestimmung, bei der ein Proband das Bild nacheinander durch verschiedene Wechseloptiken (z.B. mit verschiedener Brechkraft) betrachtet, auf einfache Weise mit Hilfe einer mobilen Computervorrichtung möglich.

Die Wechselhalterung kann insbesondere zur Aufnahme von Wechsellinsen ausgestaltet sein, und/oder in der Wechselhalterung können Wechsellinsen aufgenommen sein. Das System kann die Wechsellinsen umfassen. Hierdurch kann ein Proband das dargestellte Bild durch verschiedene Wechsellinsen betrachten, und Eigenschaften derjenigen Wechsellinse, welche den subjektiv besten Bildeindruck bietet, können dann z.B. als Grundlage für die Fertigung eines Brillenglases oder für die Auswahl einer Kontaktlinse verwendet werden.

Zusätzlich kann die Betrachtungsoptik eine Mikrolinsenanordnung umfassen. Eine Mikrolinsenanordnung ist dabei eine Anordnung einer Vielzahl von Mikrolinsen (d.h. kleiner Linsen, welche beispielsweise in einer zweidimensionalen Anordnung in Zeilen und Spalten bereitgestellt sein können.

Die Mikrolinsenanordnung kann insbesondere eine auf der Anzeige der mobilen Computervorrichtung anzubringende Mikrolinsenfolie umfassen. Damit ist ein kompakter Aufbau möglich.

Die Betrachtungsoptik kann auch einen Farbfilter umfassen. Dabei kann der Farbfilter einen Rotfilter und/oder Infrarotfilter umfassen. Hierdurch kann erreicht werden, dass von dem angezeigten Bild nur Licht der durch den Filter ausgewählten Wellenlängen das Auge erreicht. Insbesondere Rotlicht oder Infrarotlicht hat dabei einen geringeren Einfluss auf die Adaption der Pupille, so dass z.B. mit vergleichsweise hoher Lichtintensität und trotzdem vergleichsweise großer Pupille gearbeitet werden kann.

Das System kann weiter einen Strahlteiler umfassen, welcher zwischen der Anzeige der mobilen Computervorrichtung und dem mindestens einen Auge angeordnet ist, wobei der Strahlteiler angeordnet ist, von dem Auge ausgehendes Licht zu einer Kameraeinrichtung zu lenken.

Das System kann weiter, für mindestens ein Auge, eine Kombination aus Polarisator und Analysator umfassen. Hierdurch kann für das mindestens eine Auge Fremdlicht, z.B. aus einem Kanal für ein anderes Auge, unterdrückt werden.

Das System kann weiter, für mindestens ein Auge, einen Shutter umfassen.

Somit ist erfindungsgemäß mittels einer mobilen Computervorrichtung, insbesondere einem Smartphone oder einem Tablet, sowohl eine objektive Refraktionsmessung als auch eine subjektive Refraktionsmessung möglich.

Ausführungsbeispiele der vorliegenden Erfindung werden nachfolgend unter Bezugnahme auf die beigefügten Zeichnungen näher erläutert. Es zeigen:
Fig. 1 ein Blockdiagramm einer mobilen Computervorrichtung,
Fig. 2 eine schematische Außenansicht einer mobilen Computervorrichtung,
Fig. 3 ein Flussdiagramm zur Veranschaulichung der Funktionsweise von mobilen Computervorrichtungen,
Fig. 4 eine Darstellung einer möglichen strukturierten Beleuchtung zur Durchführung einer objektiven Refraktionsbestimmung,
Fig. 5 eine Alternative zu der strukturierten Beleuchtung der Fig. 5,
Fig. 6 bis 12 verschiedene Varianten von Systemen zur objektiven Refraktionsbestimmung gemäß verschiedenen Ausführungsbeispielen,
Fig. 13 ein Blockdiagramm zur Veranschaulichung der Funktionsweise von Systemen zur objektiven Refraktionsbestimmung gemäß einem Ausführungsbeispiel,
Fig. 14 bis 16 Systeme zur subjektiven Refraktionsbestimmung gemäß verschiedenen Ausführungsbeispielen, und
Fig. 17A bis 17D Beispiele für Bildschirmdarstellungen zur subjektiven Refraktionsbestimmung gemäß Ausführungsbeispielen.

Im Folgenden werden verschiedene Ausführungsbeispiele der vorliegenden Erfindung unter Bezugnahme auf die beigefügten Zeichnungen detailliert erläutert. Diese Ausführungsbeispiele dienen lediglich der Veranschaulichung und sind nicht als einschränkend auszulegen. Beispielsweise bedeutet eine Beschreibung eines Ausführungsbeispiels mit einer Vielzahl von Elementen oder Merkmalen nicht, dass alle diese Elemente oder Merkmale zur Implementierung von Ausführungsbeispielen notwendig sind. Vielmehr können andere Ausführungsbeispiele weniger Merkmale oder Elemente, alternative Merkmale oder Elemente und/oder zusätzliche Merkmale oder Elemente aufweisen. Zudem können Merkmale oder Elemente verschiedener Ausführungsbeispiele miteinander kombiniert werden, sofern nichts anderes angegeben ist.

Zunächst werden mobile Computervorrichtungen beschrieben, welche in den später beschriebenen Systemen, die Ausführungsbeispiele darstellen, verwendbar sind.

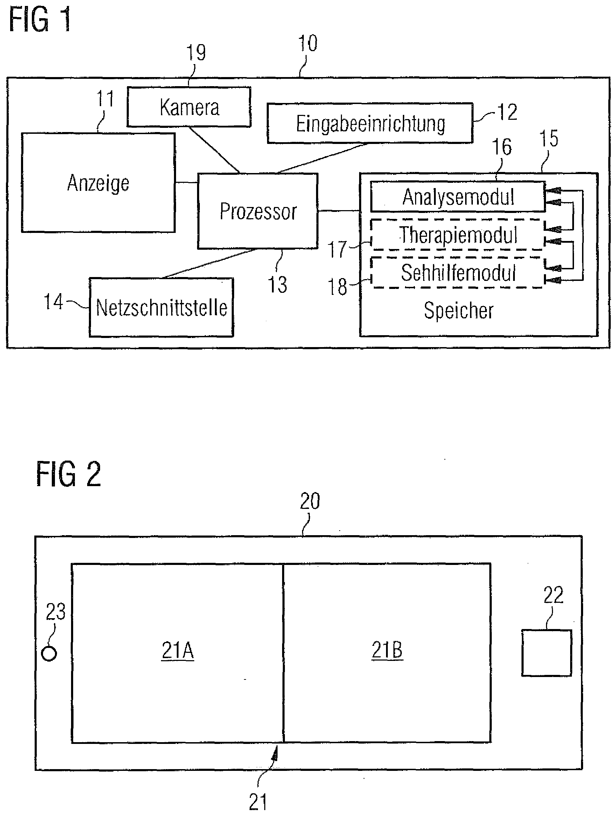

In Fig. 1 ist eine mobile Computervorrichtung 10 dargestellt. Die mobile Computervorrichtung 10 kann beispielsweise mittels eines Smartphones, eines Tablet-Computers oder mittels einer anderen mobilen Computervorrichtung (beispielsweise einem mobilen Spielgerät) implementiert sein. Derartige mobile Computervorrichtungen, welche als Grundlage für die Implementierung der mobilen Computervorrichtung 10 der Fig. 1 dienen können, sind häufig frei programmierbar, verfügen über einen Prozessor, eine Anzeige (Display, gegebenenfalls berührungsempfindlich), verschiedene Eingabeeinrichtungen, Netzschnittstellen etc. Wie im Folgenden näher erläutert wird, werden bei Ausführungsbeispielen der vorliegenden Erfindung derartige mobile Computervorrichtungen genutzt, um Möglichkeiten zur Refraktionsbestimmung bereitzustellen. Hierzu kann insbesondere die mobile Computervorrichtung entsprechend programmiert werden, beispielsweise mittels einer oder mehrerer sogenannter Apps (vom Englischen "applications", d.h. Anwenderprogramme).

Als Beispiel umfasst die mobile Computervorrichtung der Fig. 1 einen Prozessor 13, beispielsweise eine CPU. Bei anderen Varianten können auch mehrere Prozessoren oder ein Prozessor mit mehreren Prozessorkernen bereitgestellt sein. Der Prozessor 13 ist mit einem Speicher 15, beispielsweise einem Speicher mit wahlfreiem Zugriff (RAM) oder einem nichtflüchtigen Speicher wie einem Flashspeicher gekoppelt, oder Kombinationen hiervon. In dem Speicher 15 können Daten sowie Programme zum Betreiben des Prozessors 13 und der mobilen Computervorrichtung 10 abgelegt sein. Insbesondere können verschiedene Anwendungsprogramme (Apps) in dem Speicher 15 abgelegt sein, beispielsweise Anwendungsprogrammmodule 16 und 17, welche später näher diskutiert werden.

Der Prozessor 13 ist weiter mit einer Anzeige 11 gekoppelt, über welche Bilder, z.B. Informationen, Grafiken, Zeichen, Symbole und dergleichen zum Betrachten durch einen Benutzer dargestellt werden können. Der Bergriff "Bild" wird hier allgemein benutzt, um Inhalte zu bezeichnen, welche auf einer Anzeige dargestellt werden. Ein derartiges Bild kann beispielsweise Zeichen, Symbole und/oder andere Elemente umfassen. Des Weiteren ist der Prozessor 13 mit einer Eingabeeinrichtung 12 gekoppelt. Bei manchen Implementierungen kann die Anzeige 11 berührungsempfindlich sein und somit gleichzeitig die Eingabeeinrichtung 12 oder einen Teil hievon darstellen. Zusätzlich oder alternativ kann die Eingabeeinrichtung Knöpfe, Drehregler, eine Tastatur, ein Mikrofon zum Empfangen von Geräuschen oder einer Spracheingabe und/oder Sensoren wie einen Neigungssensor oder einen Beschleunigungssensor umfassen.

Des Weiteren weist bei der Fig. 1 die mobile Computervorrichtung 10 eine oder mehrere Kameras 19 auf. Beispielsweise weisen moderne Smartphones typischerweise zumindest eine Frontkamera, welche sich auf der gleichen Seite wie die Anzeige 11 befindet, sowie eine rückseitige Kamera auf der gegenüberliegenden Seite auf, wobei in vielen Fällen die rückseitige Kamera eine höhere Auflösung bietet. Es sind jedoch auch andere Konfigurationen möglich. Bei manchen Varianten wird eine derartige Kamera, wie die Kamera 19, verwendet, ein Bild eines oder beider Augen eines Benutzers aufzunehmen, um auf Basis hierauf eine objektive Refraktionsbestimmung durchzuführen. Bei anderen Varianten kann hierfür eine externe Kamera dienen.

Des Weiteren umfasst die mobile Computervorrichtung 10 der Fig. 1 eine Netzschnittstelle 14, mittels der die Computervorrichtung 10 mit weiteren Vorrichtungen kommunizieren kann. Die Netzschnittstelle 14 kann beispielsweise eine Mobilfunkschnittstelle zur Kommunikation über ein Mobilfunknetz, eine Bluetooth-Schnittstelle und/oder eine Wifi/WLAN-Schnittstelle umfassen, ist jedoch nicht hierauf beschränkt. Eine derartige Netzschnittstelle 14 kann beispielsweise zur Kommunikation mit einer oder mehreren externen Kamera(s) dienen, beispielsweise über Bluetooth. Eine derartige externe Kamera kann, wie später näher erläutert werden wird, ebenfalls zur Aufnahme eines oder beider Augen eines Benutzers dienen. Zu bemerken ist, dass die mobile Computervorrichtung 10 weitere herkömmliche Komponenten von mobilen Computervorrichtungen wie beispielsweise Smartphones aufweisen kann.

Bei der dargestellten Computervorrichtung sind in dem Speicher 15 als Anwendungsprogramme ein Analysemodul 16 zur Refraktionsbestimmung und optional zusätzlich ein weiteres Modul 17 abgelegt.

Wie durch Pfeile angedeutet können die Module auch miteinander interagieren, insbesondere Daten austauschen. Das Analysemodul 16 dient dazu, mittels der mobilen Computervorrichtung eine Refraktionsbestimmung durchzuführen. Beispielsweise können zur objektiven Refraktionsbestimmung mit einer internen Kamera wie der Kamera 19 der Fig. 1 oder einer mit der mobilen Computervorrichtung gekoppelten externen Kamera Bilder eines oder beider Augen eines Benutzers aufgenommen werden, und die Bilder können ausgewertet werden. Zusätzlich kann hierzu die Anzeige wie die Anzeige 11 angesteuert werden, ein oder mehrere Bilder, welche eine Beleuchtung für das Auge oder die Augen, insbesondere eine strukturierte Beleuchtung bilden, darzustellen. Eine strukturierte Beleuchtung kann dabei insbesondere eine Beleuchtung mit einem vorgegeben, gegebenenfalls veränderlichen, Muster sein.

Zudem kann die Anzeige 11 angesteuert werden, um Bilder, z.B. Symbole, beispielsweise Zeichen oder Zahlen, zur Durchführung einer subjektiven Refraktionsbestimmung anzuzeigen. Dies kann bei manchen Ausführungsbeispielen mit variabler Hintergrundhelligkeit erfolgen.

Zudem kann optional ein weiteres Modul 17 bereitgestellt sein, welches mit dem Analysemodul Daten austauschen kann. Das weitere Modul 17 kann beispielsweise Bestellfunktionen für Sehhilfen auf Basis der bestimmten Augenrefraktion oder gegebenenfalls auch Therapie- und/oder Sehhilfefunktionen bereitstellen.

Zudem ist über das weitere Modul 17 beispielsweise auch eine Kommunikation der Ergebnisse über die Netzschnittstelle 14 beispielsweise zu einem Augenarzt oder anderem medizinischen Fachpersonal möglich.

Es ist zu bemerken, dass das Analysemodul 16 bei manchen Ausführungsbeispielen auch nur Teilaufgaben der Refraktionsbestimmung übernehmen kann. Beispielsweise können auch Rohdaten an eine weitere Computervorrichtung gesendet werden, und die aufgenommenen Daten (beispielsweise Bilder) können dort ausgewertet werden.

Mit einer derartigen mobilen Computervorrichtung sind bei Ausführungsbeispielen und Messungen bei kontrollierten lichttechnischen Verhältnissen möglich. Damit lassen sich bei verschiedenen Adaptionspegeln und damit verschiedenen Pupillengrößen Refraktionsbestimmungen durchführen. Zudem weisen viele Anzeigen heutiger Smartphones oder Tablets einen hohen Dynamikbereich auf. Dies ermöglicht Beleuchtungen zur Analyse mit sehr hohem Kontrastumfang. Beispielsweise weisen Anzeigen auf Basis organischer Leuchtdioden (OLED) einen Kontrastumfang von 1000:1 oder mehr auf, was für die Bereitstellung einer Beleuchtung für das Auge hilfreich sein kann.

In Fig. 2 ist ein Beispiel für eine Außenansicht einer mobilen Vorrichtung 20 dargestellt. Die mobile Vorrichtung 20 der Fig. 2 kann beispielsweise der mobilen Computervorrichtung 10 der Fig. 1 entsprechen und ist in dem Beispiel der Fig. 2 als Smartphone ausgestaltet. Je nach Art des Smartphones kann sich die Außenansicht von der in Fig. 2 dargestellten unterscheiden. Die mobile Computervorrichtung 20 weist eine Anzeige 21 auf, welche insbesondere als berührungsempfindliche Anzeige (sogenannter Touchscreen) realisiert sein kann. Zudem weist bei dem dargestellten Beispiel die mobile Computervorrichtung 20 einen Bedienknopf 22 sowie eine Kamera 23 auf. Die Kamera 23 wird auch als Frontkamera bezeichnet. Eine weitere (in Fig. 2 nicht dargestellte) Kamera kann sich auf der gegenüberliegenden Seite (Rückseite) der mobilen Computervorrichtung 20 befinden. Die mobile Computervorrichtung 20 kann weitere in Fig. 2 nicht dargestellte Elemente aufweisen, wie beispielsweise einen Kopfhörerausgang, weitere Bedienelemente, einen Lautsprecher oder ein Mikrofon.

Wie in Fig. 2 dargestellt kann die Anzeige 21 in zwei Bereiche 21A und 21B unterteilt werden. In diesem Fall kann beispielsweise der Bereich 21A zur Darstellung eines Bildes und/oder einer Beleuchtung für ein linkes Auge eines Benutzers und der Bereich 21B zur Darstellung eines Bildes und/oder Beleuchtung für ein rechtes Auge eines Benutzers genutzt werden. Wie nachfolgend näher erläutert werden wird, kann die mobile Computervorrichtung 20 in eine entsprechende Betrachtungsvorrichtung eingesetzt werden.

Durch die Aufteilung der Anzeige wie in Fig. 2 dargestellt können beispielsweise durch das Analysemodul 16 linkes und rechtes Auge getrennt getestet werden, beispielsweise indem nur in dem Bereich 21A oder nur in dem Bereich 21B etwas angezeigt wird und eine entsprechende Rückmeldung von einem Benutzer ausgewertet wird, oder es können Stereosehfähigkeiten und dergleichen getestet werden.

In den Bereichen 21A und 21B können beispielsweise Bilder für linkes und rechtes Augen entsprechend einem Pupillenabstand dargestellt werden, um eine Stereobetrachtung zu ermöglichen. Beispielsweise kann ein Abstand korrespondierender Punkte so angepasst sein, dass er in etwa dem Pupillenabstand eines erwachsenen Menschen entspricht, beispielsweise ungefähr 65 mm. Bei manchen Implementierungen kann der Pupillenabstand auch ein Parameter sein, sodass die Darstellung an einem jeweiligen Benutzer und dessen Pupillenabstand angepasst werden kann.

Mobile Computervorrichtungen wie in Fig. 1 und 2 dargestellt sind häufig vergleichsweise kostengünstig, da sie in großen Stückzahlen hergestellt werden. Dennoch sind in derartigen Vorrichtungen häufig hochwertige Anzeigen (Displays) Kameras, Sensoren etc. eingesetzt.

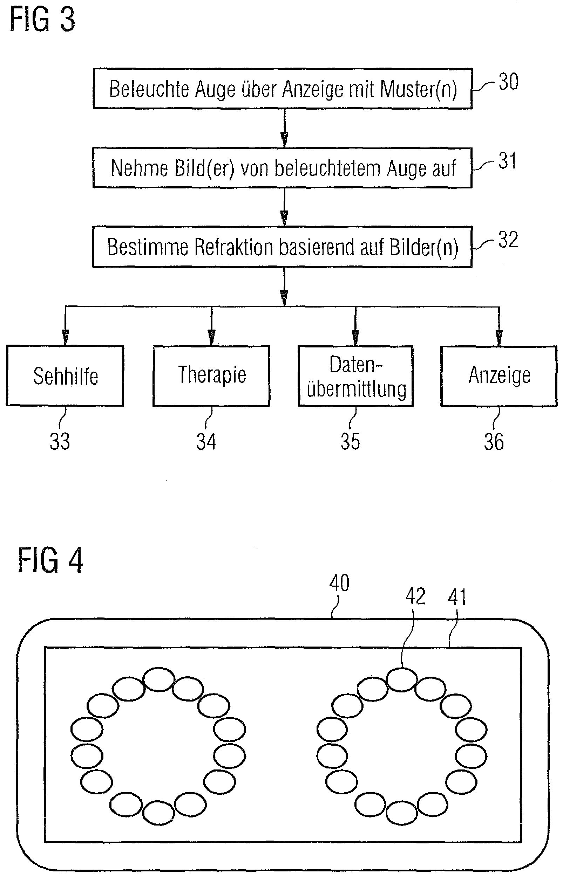

In Fig. 3 ist ein Flussdiagramm zur Veranschaulichung eines Verfahrens zur objektiven Refraktionsbestimmung dargestellt. Das Verfahren kann beispielsweise durch das Analysemodul 16 die Fig. 1 mittels einer mobilen Computervorrichtung wie der mobilen Computervorrichtung 10 der Fig. 1 implementiert sein.

Zur Vereinfachung wird dabei bei der Beschreibung der Fig. 3 nur ein Auge erwähnt. Die verschiedenen Schritte der Fig. 3 sind jedoch auf beide Augen, hintereinander oder simultan, anwendbar. "Ein" bei "ein Auge" ist also als unbestimmter Artikel und nicht als einschränkende Zahlenangabe zu verstehen.

In einem Schritt 30 wird ein Auge eines Benutzers über eine Anzeige einer mobilen Computervorrichtung (beispielsweise Anzeige 11 der Fig. 1 oder Anzeige 21 der Fig. 2) mit einem Muster, welches sich insbesondere zeitlich verändern kann, beleuchtet. In Schritt 31 werden dann entsprechend Bilder von dem beleuchteten Auge aufgenommen.

In Schritt 32 wird die Augenrefraktion basierend auf den Bildern bestimmt. Dies kann grundsätzlich wie in den in der Einleitung erwähnten Vorrichtungen, welche eine Beleuchtung aus verschiedenen Richtungen benutzen, geschehen, wobei bei dem Ausführungsbeispiel der Fig. 3 die Anzeige des Smartphones als Beleuchtung dient.

Die so bestimmte Augenrefraktion kann auf verschiedene Weise verwendet werden, wie beispielhaft mit Schritt 33 bis 36 der Fig. 3 angedeutet. Die Schritte 33 bis 36 können unabhängig voneinander implementiert sein und können bei anderen Ausführungsbeispielen auch ganz oder teilweise weggelassen sein.

Beispielsweise wird bei 33 die bestimmte Augenrefraktion verwendet, um eine Sehhilfe (beispielsweise Brillen oder Kontaktlinsen) entsprechend anzupassen. Bei 34 wird die bestimmte Refraktion als Grundlage für eine Therapie verwendet. Bei 35 erfolgt eine Datenübermittlung, beispielsweise zu medizinischem Fachpersonal wie einem Arzt oder einem Optiker. Beispielsweise können, wenn die Refraktion bestimmt würde, entsprechende Daten an einen Optiker übermittelt werden, welcher dann eine entsprechende Brille bereitstellen kann. Eine Datenübermittlung ist beispielsweise auch direkt an einem Brillenhersteller oder Brillenglashersteller möglich, welcher dann entsprechende Brillen oder Brillengläser bereitstellen kann. Vor einer derartigen Bestellung kann eine entsprechende Rückfrage an einen Benutzer und/oder medizinisches Fachperson erfolgen. Schließlich kann die bestimmte Augenrefraktion auch einfach bei 36 angezeigt werden, oder es kann basierend auf der Augenrefraktion auf einer Anzeige eine Empfehlung ausgesprochen werden, beispielsweise eine Empfehlung, einen Augenarzt aufzusuchen.

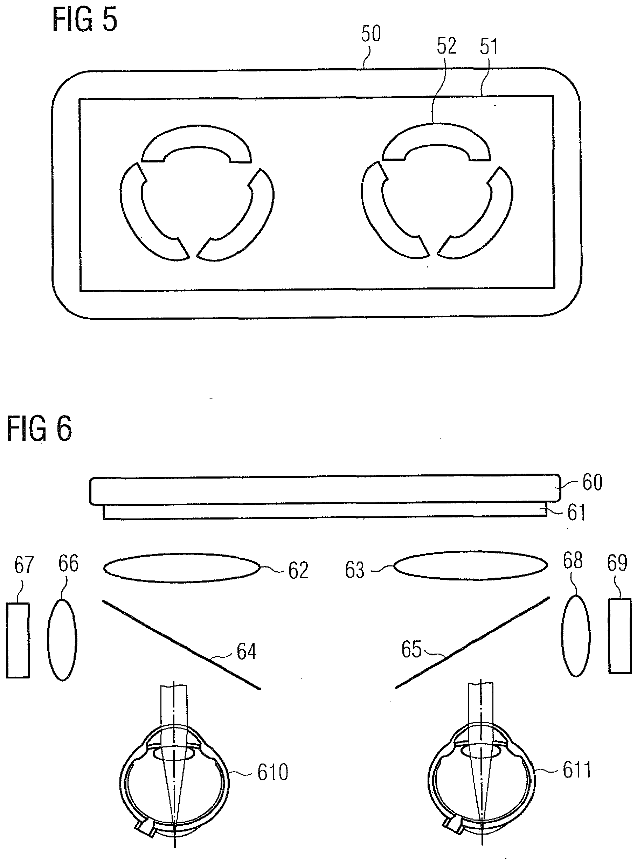

Wie bereits erläutert, kann zur Bestimmung der Refraktion ein Auge (oder beide Augen) eines Benutzers, d.h. einer zu untersuchenden Person, strukturiert beleuchtet werden. Insbesondere kann eine Beleuchtung sequenziell aus verschiedenen Richtungen erfolgen, ähnlich dem eingangs diskutierten Stand der Technik, wobei bei den dargestellten Computervorrichtungen im Gegensatz zu dem diskutierten Stand der Technik die Beleuchtung mithilfe einer Anzeige einer mobilen Computervorrichtung, wie beispielsweise einem Smartphone, und nicht mittels diskreter Lichtquellen wie Leuchtdioden erfolgt. Beispiele einer derartigen Beleuchtung werden nunmehr unter Bezugnahme auf die Fig. 4 und 5 näher erläutert.

Als erstes Beispiel ist in Fig. 4 eine mobile Computervorrichtung 40, beispielsweise ein Smartphone oder ein Tablet-Computer, mit einer Anzeige (Display) 41 dargestellt. In Fig. 4 können auf der Anzeige 41 getrennt für linkes und rechtes Auge Lichtquellenpunkte 42, welche jeweils eine einstellbare Ringbeleuchtung bilden, um das jeweilige Auge aus verschiedenen Richtungen zu beleuchten, dargestellt werden. Dabei könnten die einzelnen Lichtquellenpunkte 42 beispielsweise sequenziell oder auch in Gruppen aktiviert werden, um so je nach aktiviertem Lichtquellenpunkt 42 das Auge aus einer jeweiligen Richtung zu beleuchten. Der Leuchtdichtepegel der Lichtquellenpunkte 42 kann dabei einstellbar sein, d.h. die Helligkeit der Lichtquellenpunkte 42 kann je nach Bedarf innerhalb der Möglichkeiten, die die Anzeige 41 bietet, veränderbar sein. Auch die Farbe kann einstellbar sein.

Zudem kann auch eine Umfeldleuchtdichte L_u einstellbar sein, d.h. der Hintergrund (der Teil der Anzeige 41, welcher nicht dazu dient, eine gerade aktive Punktwelle 42 darzustellen) kann ebenso einstellbar sein. Die Lichtquellenpunkte 42 in Fig. 4 sind dabei z.B. Kreise einer bestimmten Größe, also keine Punkte im mathematischen Sinn, und können näherungsweise als Punktlichtquellen dienen.

Die Beleuchtung mit Lichtquellenpunkten der Fig. 4 dient lediglich als Beispiel, und es sind auch andere Beleuchtungen möglich. So zeigt Fig. 5 eine weitere Möglichkeit. Hier werden auf einer Anzeige 51 einer mobilen Computervorrichtung 50 Ringsegmente 52 angezeigt, welche wahlweise einzeln oder in Gruppen aktivierbar und deaktivierbar sind. Wie bei dem Ausführungsbeispiel der Fig. 4 können auch bei Fig. 5 sowohl eine Leuchtdichte der Kreissegmente 52 als auch eine Hintergrundleuchtdichte (Umfeldleuchtdichte) einstellbar sein.

Auch andere Formen sind möglich. Beispielsweise können konzentrische Ringe verschiedener Größen ebenso als strukturierte Beleuchtung dienen, oder einzelne Punkte, welche sich auch auf der Anzeige bewegen können. In den Fig. 4 und 5 sind jeweils Punktwellen 42 und Ringsegmente 52 für linkes und rechtes Auge dargestellt. In diesem Fall ist die Anzeige wie unter Bezugnahme auf Fig. 2 erläutert geteilt. Bei anderen Implementierungen kann die Anzeige auch nur zur Darstellung einer Beleuchtung für ein Auge verwendet werden. In diesem Fall kann beispielsweise die Position der mobilen Computervorrichtung 50 je nach zu untersuchendem Auge verändert werden.

Durch Veränderung der Leuchtdichten kann insbesondere skotopisches Sehen (auch als Nachtsehen oder Stäbchensehen bezeichnet), photopisches Sehen (auch als Tagsehen oder Zapfensehen bezeichnet) sowie der Übergangsbereich hierzwischen (mesopisches Sehen oder Dämmerungssehen) getrennt untersucht werden.

Als nächstes werden verschiedene Systeme diskutiert, bei welchen mittels einer mobilen Computervorrichtung wie einem Smartphone, eine objektive Refraktionsbestimmung durchführbar ist. Verschiedene Varianten dieser Systeme werden unter Bezugnahme auf die Fig. 6 bis 12 erläutert. Um Wiederholungen zu vermeiden, sind dabei gleiche oder einander entsprechenden Elemente mit den gleichen Bezugszeichen gekennzeichnet und werden nicht wiederholt erläutert.

Ein erstes Ausführungsbeispiel ist in Fig. 6 dargestellt. Dabei umfasst das System der Fig. 6 eine mobile Computervorrichtung 60, z.B. ein Smartphone oder Tablet, mit einer Anzeige 61, welches zum Darstellen einer strukturierten Beleuchtung für ein linkes Auge 610 und ein rechtes Auge 611 eines Benutzers dient. Die mobile Computervorrichtung 60 kann dabei wie unter Bezugnahme auf die Fig. 1 und 2 diskutiert ausgestaltet sein, die unter Bezugnahme auf Fig. 3 diskutierten Funktionen ausführen und/oder die unter Bezugnahme auf die Fig. 4 und 5 diskutierte Beleuchtung bereitstellen.

In Fig. 6 ist die mobile Computervorrichtung 60 beispielsweise in einer Halterung bereitgestellt, in welcher auch die übrigen in Fig. 6 dargestellten Komponenten bereitgestellt sind bzw. mit dieser gekoppelt sind. Eine derartige Halterung kann beispielsweise am Kopf getragen werden (beispielsweise als sogenanntes Head-Mounted Display, HMD).

Bei dem System der Fig. 6 umfasst das System weiter Optiken 62, 63, um ein auf der Anzeige 61 dargestelltes Bild zu dem linken Auge 610 bzw. dem rechten Auge 611 abzubilden. Die Optiken 62, 63 können, wie oben diskutiert, beispielsweise in einer Betrachtungsvorrichtung wie einem Head-Mounted Display bereitgestellt sein. Während die Optiken 62, 63 zur Vereinfachung in Fig. 6 als einzelne Linsen dargestellt sind, können die Optiken 62, 63 auch jeweils mehrere optische Elemente, beispielsweise Linsen, umfassen.

Zur Aufnahme eines Bildes des linken Auges 610 ist eine erste Kameraeinrichtung mit einem Bildsensor 67, beispielsweise ein CMOS-Sensor oder ein CCD-Sensor, und mit einer Kameraoptik 66 bereitgestellt. Zur Aufnahme eines Bildes des rechten Auges ist in entsprechender Weise eine zweite Kameraeinrichtung mit einem Bildsensor 69 und mit einer Kameraoptik 68 bereitgestellt. Auch die Kameraoptiken 66, 68 können eine oder mehrere Linsen oder andere optische Elemente umfassen.

Zur Bildaufnahme weist die Vorrichtung der Fig. 6 Strahlteiler 64, 65 auf. Mittels des Strahlteilers 64, 65 wird das Licht von den Augen 610, 611 zu den Bildsensoren 67 bzw. 69 gelenkt. Licht von der Anzeige 61 gelangt durch die Strahlteiler 64, 65 hindurch zu den Augen 610, 611. Die Strahlteiler 64, 65 können beispielsweise teildurchlässige Spiegel sein.

Die ersten und zweiten Kameraeinrichtungen können bei manchen Implementierungen mit der mobilen Computervorrichtung 60 gekoppelt sein, beispielsweise drahtlos (beispielsweise über Bluetooth) oder auch drahtgebunden (beispielsweise über einen USB-Anschluss oder eine andere Verbindung). Bei wieder anderen Ausführungsbeispielen können sowohl die ersten und zweiten Kameraeinrichtungen als auch die mobile Computervorrichtung 60 von einer zusätzlichen (nicht dargestellten) Vorrichtung gesteuert werden.

Durch das Bereitstellen zweier Kameraeinrichtungen 66-69 kann neben der objektiven Refraktionsbestimmung für die Augen 610, 611 auch eine Untersuchung des stereoskopischen Sehens (beide Augen gemeinsam) durchgeführt werden.

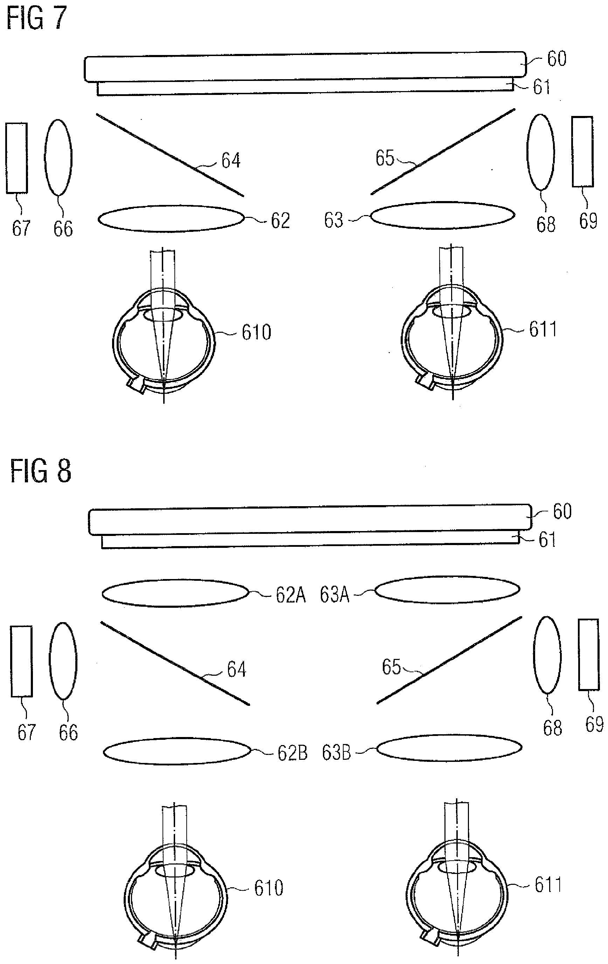

Als nächstes werden Variationen des Systems unter Bezugnahme auf die Fig. 7 bis 12 beschrieben. Dabei werden, wie bereits erläutert, im Wesentlichen die Unterschiede zur Fig. 6 oder zu anderen vorher beschriebenen Figuren diskutiert, und Elemente, welche gleich bleiben, werden nicht nochmals diskutiert.

In Fig. 7 ist verglichen mit Fig. 6 die Position der Strahlteiler 64, 65 mit der Position der Optiken 62, 63 vertauscht. In Fig. 8 sind die Optiken 62A, 62B für die Optik 62 und in Optiken 63A, 63B für die Optik 63 zweigeteilt. Der Strahlteiler 64 ist zwischen den Optiken 62A und 62B angeordnet, und der Strahlenteiler 65 ist zwischen den Optiken 63A und 63B angeordnet. Bei manchen Ausführungsbeispielen können die Strahlteiler 64 und 65 auch innerhalb von Linsen der Optiken 62, 63 angeordnet sein. In anderen Worten zeigen die Fig. 6 bis 8, dass die Anordnung der Strahlteiler 64, 65 und der Optiken 62, 63 variabel ist.

Zu bemerken ist, dass die Optiken 62B, 63B auch austauschbar sein können, was, wie später erläutert werden wird, eine subjektive Refraktionsbestimmung ermöglichen kann.

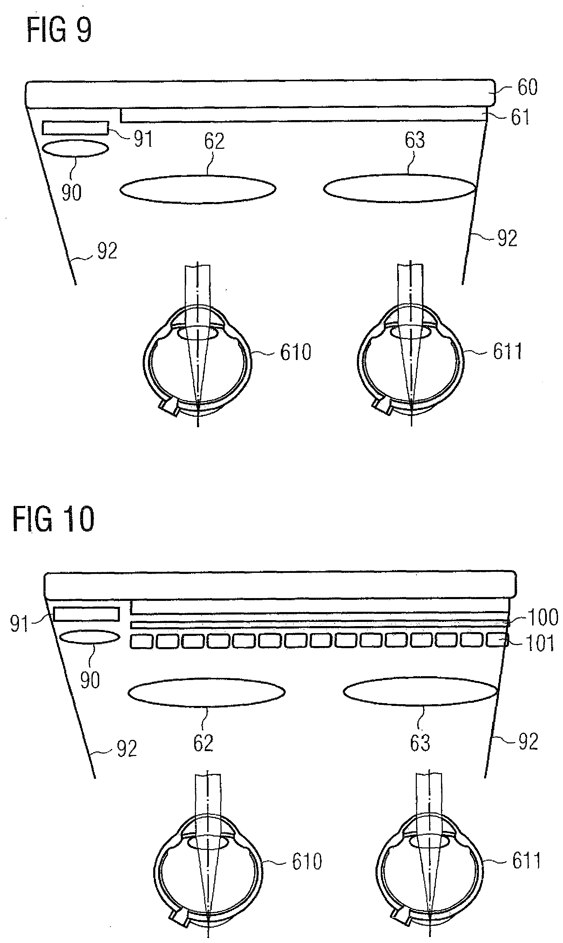

Bei den unter Bezugnahme auf die Figuren 6-8 diskutierten Systemen werden zu der mobilen Computervorrichtung 60 externe Kameraeinrichtungen (66-69) verwendet, um ein Bild der Augen 610, 611 aufzunehmen. Bei anderen Implementierungen kann hierfür eine interne Kamera der mobilen Computervorrichtung 60 verwendet werden. Eine derartige Implementierung ist in Fig. 9 dargestellt.

Bei dem System der Fig. 9 weist die mobile Computervorrichtung 60 eine Kameraeinrichtung mit einem Bildsensor 91 und einer Kameraoptik 90 auf. Der Bildsensor 91 kann beispielsweise ein CMOS-Sensor oder ein CCD-Sensor sein. Bei dem dargestellten System erfolgt wiederum über die Anzeige 61 der mobilen Computervorrichtung 60 eine strukturierte Beleuchtung der Augen, und ein oder beide Augen werden über den Bildsensor 91 aufgenommen, um somit eine objektive Refraktionsbestimmung durchführen zu können. Mit 92 ist ein Gehäuse oder dergleichen bezeichnet, welches insbesondere das Eindringen von Streulicht verhindern oder verringern kann.

Je nach Ausgestaltung der Kameraeinrichtung 90, 91 kann es sein, dass nur ein Auge (in Fig. 9 beispielsweise das Auge 610) aufgenommen werden kann. In diesem Fall muss beispielsweise zur Refraktionsbestimmung des Auges 611 die Ausrichtung der mobilen Computervorrichtung 60 umgedreht werden. Bei anderen Systemen können zusätzliche optische Elemente (beispielsweise Strahlteiler) bereitgestellt sein, um Licht vom Auge 611 zu dem Bildsensor 91 zu lenken. Bei anderen Systemen kann die mobile Computervorrichtung 60 zwei Kameras aufweisen.

Dabei wird durch Benutzung einer internen Kamera der mobilen Computervorrichtung 60 der Aufbau insofern vereinfacht, als dass keine zusätzliche externe Kameravorrichtung, ggf. verbunden mit optischen Elementen wie Strahlteilern (beispielsweise 64, 65 der Figuren 6-8) nötig sind. Auf der anderen Seite kann je nach Lage der Kamera wie oben erläutert nur ein Auge aufgenommen werden, was die Augenuntersuchung ggf. verlängern kann. Zudem ist es bei einer Kamera, die simultan nur ein Auge aufnehmen kann, nicht möglich, auch stereoskopisches Sehen zu messen.

Bei den Systemen der Figuren 6-9 werden Betrachtungsoptiken 62, 63 beispielsweise eines Headup-Displays oder einer anderen Betrachtungsvorrichtung verwendet. Derartige Betrachtungsvorrichtungen für Smartphones sind kommerziell erhältlich. Je nach Qualität der Optiken 62, 63 und der verwendeten strukturierten Beleuchtung können unter Umständen die Optiken 62, 63 unzureichend sein, um eine gewünschte Abbildung der auf der Anzeige 61 dargestellten Beleuchtung auf die Augen 610, 611 zu gewährleisten.

In diesem Fall können beispielsweise, wie in Fig. 10 dargestellt, eine zusätzliche Mikrolinsenanordnung 101 vor der Anzeige 61 bereitgestellt werden. Diese Mikrolinsenanordnung kann eine gewünschte Abbildung der auf der Anzeige 61 dargestellten Beleuchtung (beispielsweise Punktlichtquellen 42 der Fig. 2, 4 oder der Ringsegmente 52 der Fig. 5) auf die Augen 610, 611 gewährleisten. Bei einem bevorzugten Ausführungsbeispiel ist die Mikrolinsenanordnung 101 als Mikrolinsenfolie bereitgestellt, welche einfach auf die Anzeige 61 aufgeklebt oder anders aufgebracht werden kann. Auf diese Weise kann die Mikrolinsenanordnung 101 auf einfache Weise einem Endanwender bereitgestellt werden.

Zudem weist bei dem System der Fig. 10 das dargestellte System einen Filter 100 auf, welcher ebenfalls als Folie bereitgestellt werden kann und zwischen der Anzeige 61 und der Mikrolinsenanordnung 101 angeordnet ist. Auch andere Anordnungen sind möglich. Der Filter 100 kann bei manchen Ausführungsbeispielen auch mit den Mikrolinsen 101 in einer einzigen Folie bereitgestellt sein, welche dann auf die Anzeige 61 geklebt oder anders aufgebracht werden kann. Der Filter 100 kann dabei insbesondere ein Rot- und/oder Infrarotfilter sein, welcher beispielsweise rotes Licht und/oder Infrarotlicht passieren lässt. Die Verwendung von rotem Licht für die Beleuchtung der Augen 610 und 611 kann den Vorteil bieten, dass hierdurch eine geringere Verengung der Augenpupille hervorgerufen wird als beispielsweise mit weißem Licht, da die wahrgenommene Helligkeit geringer ist. Bei anderen Ausführungsbeispielen kann die Farbe der Beleuchtung direkt über die Anzeige 61 eingestellt werden.

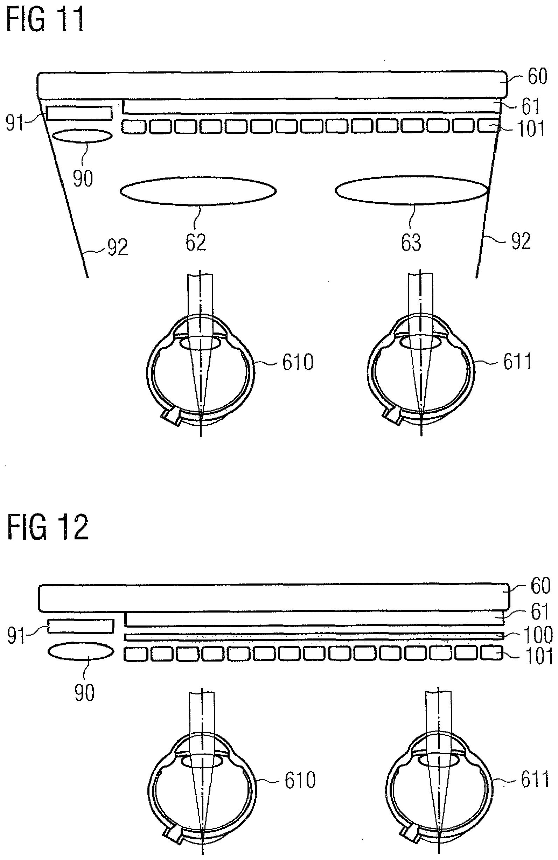

Somit kann der Filter 100 auch weggelassen werden, wie dies im System der Fig. 11 dargestellt ist. Abgesehen von dem weggelassenen Filter 100 entspricht das Ausführungsbeispiel der Fig. 11 dem Ausführungsbeispiel der Fig. 10.

Bei wieder anderen Systemen können die Mikrolinsen 101 alleine bereits ausreichend sein, um eine Abbildung einer Beleuchtung auf die Augen 610, 611 zu gewährleisten. Ein derartiges Ausführungsbeispiel ist in Fig. 12 dargestellt.

Bei dem System der Fig. 12 ist auf der Anzeige 61 lediglich der Filter 100 und die Mikrolinsen 101 bereitgestellt. Die Optik 62, 63 und das Gehäuse 92 sind hingegen weggelassen, d.h., in diesem Fall kann die mobile Computervorrichtung 60 beispielsweise ohne ein Head-Mounted-Display oder dgl. bereitgestellt werden. Dabei kann beispielsweise ein Benutzer angewiesen sein, die mobile Computervorrichtung 60 in einer festgelegten Distanz zu seinen Augen 610, 611 zu halten. Bei manchen Systemen kann die korrekte Ausrichtung zu den Augen 610, 611 auch über die Kameraeinrichtung 90, 91 kontrolliert werden. Beispielsweise kann mittels eines entsprechenden Moduls wie dem Analysemodul 16 der Fig. 1 überprüft werden, ob die Augen relativ zu dem Bildsensor 91 eine gewünschte Position haben bzw. auf dem Bildsensor 91 in einer gewünschten Größe abgebildet werden. Auch können andere Hilfsmittel, wie ein Maßstab, zur Positionierung bereitgestellt werden. Bei dem System der Fig. 12 muss einem Benutzer beispielsweise lediglich eine Folie mit dem Filter 100 und den Mikrolinsen 101 zum Aufkleben oder anderweitig Aufbringen auf die Anzeige 61 bereitgestellt werden. Bei anderen Ausführungsbeispielen kann der Filter 100 auch weggelassen sein.

Es ist zu bemerken, dass Filter 100 und/oder Mikrolinsenfolie 101 auch bei den Systemen der Figuren 6-9 bereitgestellt werden können.

Wie aus den Figuren 6-12 ersichtlich, sind also verschiedene Systeme und Aufbauten möglich, um eine objektive Refraktionsbestimmung mittels einer mobilen Computervorrichtung wie einem Smartphone oder einem Tablet zu erhalten. Dabei können zusätzlich noch andere Messungen, wie beispielsweise allgemein ein Seh-/Visustest, insbesondere auch ein stereoskopischer Test, durchgeführt werden.

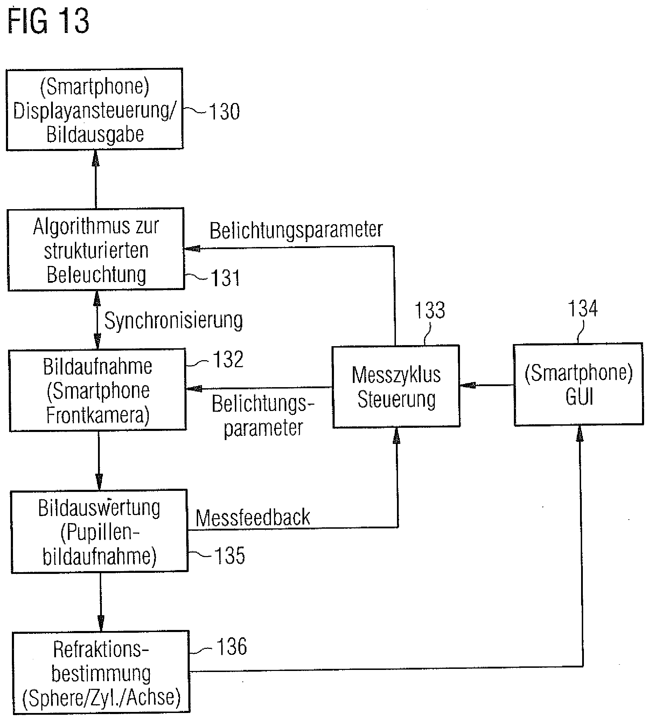

In Fig. 13 ist eine mögliche Funktionsweise derartiger Systeme zur Refraktionsbestimmung nochmals dargestellt. Bei der Darstellung der Fig. 13 wird ein Smartphone als Beispiel für eine mobile Computervorrichtung verwendet, es können jedoch auch andere mobile Computervorrichtungen, beispielsweise Tablet-Computer oder mobile Spielgeräte, verwendet werden. Die verschiedenen unter Bezugnahme auf Fig. 13 im Folgenden erläuterten Funktionalitäten können beispielsweise mittels der vorstehend beschriebenen Vorrichtungen und Systeme implementiert sein, beispielsweise mittels einer entsprechenden Applikation auf einem Smartphone. Somit sind die verschiedenen Blöcke der Fig. 13 nicht notwendigerweise als getrennte Einheiten implementiert, sondern manche der Blöcke können auch gemeinsam mittels einer Applikation auf einer mobilen Computervorrichtung implementiert sein.

Die Bedienung erfolgt dabei wie durch einen Block 134 dargestellt über eine Benutzerschnittstelle der mobilen Computervorrichtung, beispielsweise des Smartphones. Eine derartige Benutzerschnittstelle kann insbesondere eine grafische Benutzerschnittstelle sein, auch als GUI, vom englischen Graphical User Interface, bezeichnet. Hierzu kann insbesondere eine berührungsempfindliche Anzeige (so genannter Touchscreen) verwendet werden. Mittels der Benutzerschnittstelle 134 kann insbesondere eine Messzyklussteuerung 133, welche die Refraktionsbestimmung steuert, gestartet und/oder eingestellt werden.

Die Messzyklussteuerung 133 übergibt Beleuchtungsparameter an einen Algorithmus zur strukturierten Beleuchtung 131, welcher wiederum eine Anzeige, beispielsweise ein Display, der mobilen Computervorrichtung bei 130 ansteuert, um eine gewünschte Beleuchtung zu erzielen. Beispielsweise kann eine Beleuchtung wie unter Bezugnahme auf Fig. 4 oder 5 diskutiert auf diese Weise realisiert werden. Die Beleuchtungsparameter können dabei beispielsweise eine gewünschte Hintergrundhelligkeit, eine gewünschte Art der Beleuchtung (beispielsweise wie in Fig. 4 oder in Fig. 5 dargestellt) und dgl. umfassen. Zudem steuert die Messzyklussteuerung 133 eine Bildaufnahme 132, beispielsweise mittels einer Frontkamera eines Smartphones wie in den Figuren 9-12 dargestellt oder auch mit einer oder mehreren externen Kameras wie in den Figuren 6-8 dargestellt. Dabei kann die Messzyklussteuerung 133 insbesondere Belichtungsparameter, beispielsweise Belichtungszeit, Blende und/oder Isowert, einstellen. Die strukturierte Beleuchtung 131 ist dabei bei dem Ausführungsbeispiel der Fig. 13 mit der Bildaufnahme 132 synchronisiert. Beispielsweise kann für jede Beleuchtungsrichtung (beispielsweise jede Punktlichtquelle 42 der Fig. 4 und/oder jedes Kreissegment 52 der der Fig. 5) ein Bild (oder mehrere Bilder) aufgenommen werden.

Bei 135 erfolgt dann eine Bildauswertung des bei 132 aufgenommenen Bildes. Dabei kann insbesondere ausgewertet werden, ob die Aufnahme einer oder beider Pupillen korrekt erfolgte. Basierend darauf kann der Messzyklussteuerung 133 ein Messfeedback gegeben werden. Basierend auf dem Messfeedback können dann die Beleuchtungsparameter und/oder die Belichtungsparameter angepasst werden. Beispielsweise kann bei einem verwackelten Bild die Belichtungszeit verkürzt werden, oder eine Beleuchtungsstärke kann erhöht werden, wenn ein auszuwertender Pupillenreflex nicht sichtbar ist.

Bei 136 erfolgt dann eine Refraktionsbestimmung auf Basis der aufgenommenen Bilder.

Dabei kann insbesondere der Pupillenrückreflex (optischer Durchgang des Lichtes durch die Augenlinse gefolgt von einer Reflexion an der Retina und einem zweiten Durchgang durch die Augenlinse) ausgewertet werden. Durch Auswertung des Gradientenverlaufes im Reflexbild bei wechselnder Beleuchtungsrichtung kann dann die Fehlsichtigkeit des Auges (Sphäre, Zylinder, Achse) bestimmt werden, da das Verhalten des Rückreflexes von diesen Parametern abhängt.

Dabei können Systeme wie oben beschrieben dadurch, dass die mobile Computervorrichtung relativ nahe an dem zu vermessenden Auge oder den zu vermessenden Augen angeordnet sind, ein großes Gesichtsfeld erreicht werden, d.h. die Anzeige der mobilen Computervorrichtung kann bei den dargestellten Systemen einen vergleichsweise großen Teil des Gesichtsfeldes, beispielsweise mehr als 50% oder mehr als 70%, ausfüllen. Hierdurch kann die "Hintergrundbeleuchtung" entsprechend einem gewünschten Leuchtdichteadaptionspegel eingestellt werden sowie ein beliebiges Beleuchtungsmuster für die Refraktionsbestimmung eingestellt werden. Somit kann die optische Refraktionsbestimmung mittels Photorefraktion bei unterschiedlichen Pupillengrößen entsprechend den unterschiedlichen Umfeldleuchtdichten durchgeführt werden. Insbesondere bei Systemen, in welchen der Abstand der mobilen Computervorrichtung zu dem Auge oder den Augen klar definiert ist (beispielsweise bei Verwendung eines Headup-Displays) ist im Gegensatz zu herkömmlichen Handheld-Fotorefraktionmetern keine Entfernungsmessung notwendig.

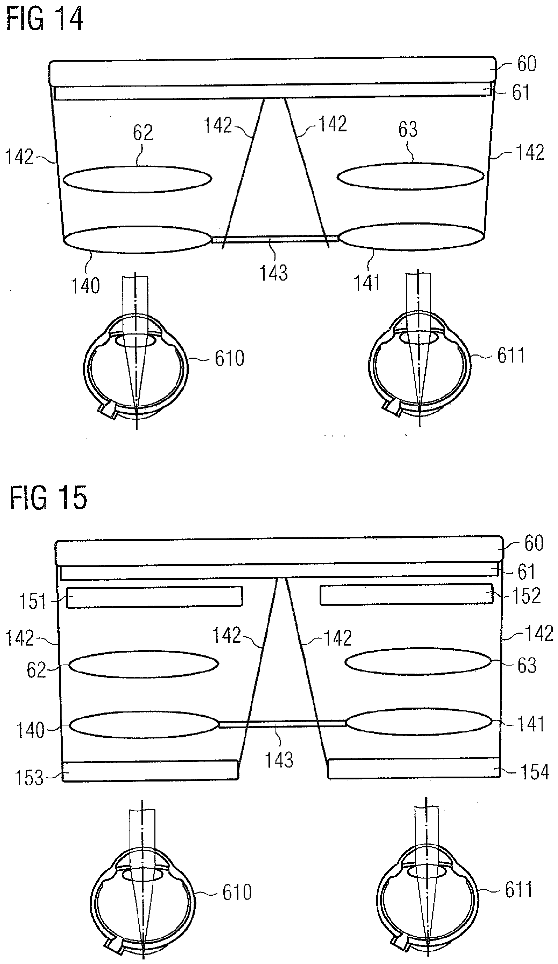

Wie bereits erläutert erfolgt bei den unter Bezugnahme auf die Figuren 3-13 diskutierten Systemen eine objektive Refraktionsbestimmung durchgeführt. Wie eingangs bereits erläutert können mit Ausführungsbeispielen der Erfindung zusätzlich oder alternativ auch subjektive Refraktionsbestimmungen durchgeführt werden. Derartige Ausführungsbeispiele werden nunmehr unter Bezugnahme auf die Figuren 14-17 erläutert. Elemente der Figuren 14-16, welche Elementen der Figuren 6-12 entsprechen, tragen wiederum die gleichen Bezugszeichen und werden nicht nochmals detailliert erläutert. Insbesondere bezeichnet 60 wiederum eine mobile Computervorrichtung wie ein Smartphone mit einer Anzeige 61, und 610 und 611 bezeichnen zwei Augen einer zu untersuchenden Person. Mit 62 und 63 sind Betrachtungsoptiken einer Betrachtungsvorrichtung wie einem Head-Mounted-Display oder dgl. bezeichnet.

Mit 140 und 141 sind Wechsellinsen oder andere Wechseloptiken bezeichnet, d.h. austauschbare Linsen oder andere Optiken. Mit 143 ist eine Halterung für die austauschbaren Linsen 140, 141, beispielsweise ein Wechselrahmen, bezeichnet. Mit 142 sind Gehäuseteile oder andere Abschattungen bezeichnet, welche zum einen Umgebungslicht abschatten und zum anderen für das linke Auge 610 und das rechte Auge 611 getrennte Lichtkanäle bereitstellen. Zur subjektiven Refraktionsbestimmung werden dann auf der Anzeige 61 Zeichen, Symbole oder Bilder dargestellt, und die zu untersuchende Person gibt an, ob sich bei einem Wechsel der Linsen 140, 141 beispielsweise der Bildeindruck verbessert oder verschlechtert, und/oder ob die Person mehr oder weniger Symbole oder Zeichen erkennen kann.

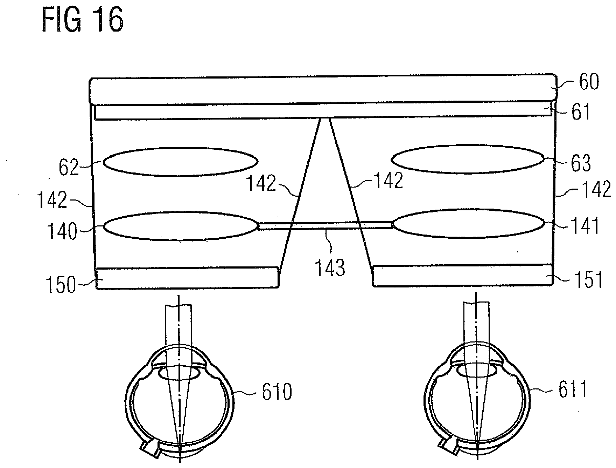

Wie bereits erläutert können bei manchen Ausführungsbeispielen sowohl eine objektive Refraktionsbestimmung als auch eine subjektive Refraktionsbestimmung durchgeführt werden. Bei derartigen kombinierten Systemen können beispielsweise die Linsen 62B und 63B der Fig. 8 den Linsen 140, 141 der Fig. 14 entsprechen. Die subjektive Refraktionsbestimmung kann dabei insbesondere getrennt für linkes Auge 610 und rechtes Auge 611 durchgeführt werden. Es ist jedoch auch eine gemeinsame Benutzung beider Augen (für stereoskopisches Sehen) möglich. In jedem Fall ist es dabei hilfreich, wenn die Lichtwege für linkes Auge 610 und rechtes Auge 611 möglichst gut getrennt sind, damit keine gegenseitige Störung vorliegt. In anderen Worten sollte ein für das linke Auge 610 dargestellter Bildanteil (beispielsweise aus dem Bereich 21A der Fig. 2) möglichst nur zu dem linken Auge 610 gelangen, und ein für das rechte Auge 611 auf der Anzeige 61 dargestellter Bildanteil (beispielsweise aus dem Bereich 21B der Fig. 2) sollte möglichst nur zu dem rechten Auge 611 gelangen. Je nach Design kann dabei die Trennung durch das Gehäuse 142 unzureichend sein. Beispielsweise kann bei manchen Headup-Displays das Gehäuse nicht ganz bis zur Anzeige 61 reichen, beispielsweise um ein leichtes Herausnehmen der Computervorrichtung 60 aus dem Gehäuse zu ermöglichen. In derartigen Fällen können zusätzliche Maßnahmen ergriffen werden. Zwei Möglichkeiten hierzu werden unter Bezugnahme auf die Figuren 15 und 16 erläutert.

Die Systeme der Figuren 15 und 16 beruhen dabei auf dem System der Fig. 14, und gleiche Elemente tragen die gleichen Bezugszeichen und werden nicht nochmals erläutert.

Bei dem Ausführungsbeispiel der Fig. 15 sind Polarisatoren 151 und 152 über der Anzeige 61 angeordnet. Die Polarisatoren 151, 152 können zueinander senkrechte Polarisationen aufweisen (beispielsweise linkszirkular und rechtszirkular oder zwei zueinander senkrechte lineare Polarisationsrichtungen).

Zudem sind an Lichtaustrittsorten des Gehäuses 142 Analysatoren 153, 154 angeordnet. Eine Polarisationsrichtung des Analysators 153 entspricht dabei derjenigen des Polarisators 151. In ähnlicher Weise entspricht eine Polarisation des Analysators 154 einer Polarisation des Polarisators 152. Auf diese Weise kann bei manchen Ausführungsbeispielen eine verbesserte Trennung der Lichtwege für linkes und rechtes Auge erreicht werden.

Eine weitere Möglichkeit ist in Fig. 16 dargestellt. Bei dem Ausführungsbeispiel der Fig. 16 sind Shutter 160 bzw. 161 für das linke Auge 610 bzw. das rechte Auge 611 bereitgestellt. Die Shutter 160, 161 können beispielsweise LCD-Shutter sein. Durch die Shutter 160, 161 kann abwechselnd das linke und das rechte Auge abgedunkelt werden, und es kann gleichzeitig nur für das jeweils nicht abgedunkelte Auge ein Bild auf der Anzeige 61 (beispielsweise in dem entsprechenden Bereich 21A oder 21B der Fig. 2) dargestellt werden. Bei manchen Ausführungsbeispielen kann dabei der Wechsel zwischen linkem Auge und rechtem Auge so schnell erfolgen, dass für beide Augen gleichzeitig ein Bild dargestellt wird. Auch auf diese Weise kann die Trennung der Lichtwege für das linke Auge 610 und das rechte Auge 611 verbessert werden.

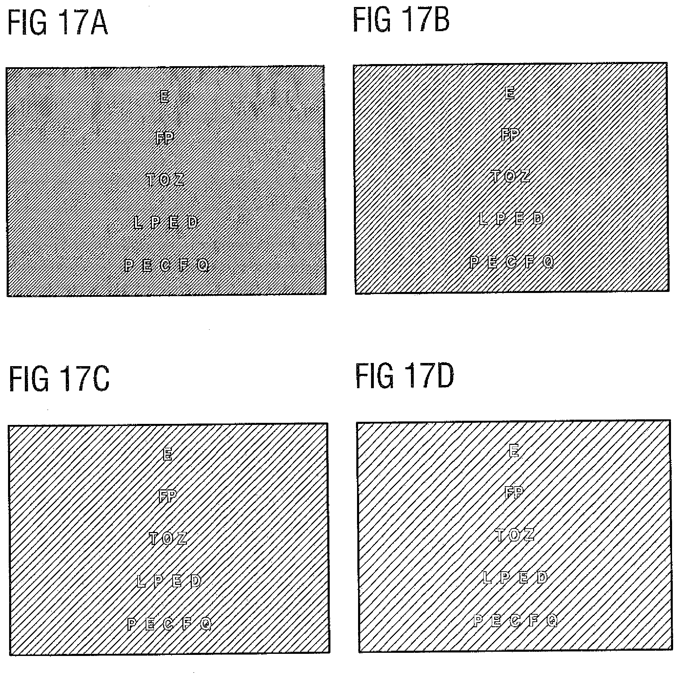

In den Figuren 17A-17D sind verschiedene Beispiele für Buchstaben, welche zur Durchführung einer subjektiven Refraktionsbestimmung angezeigt werden können, dargestellt. Das Größenverhältnis Buchstaben zu Hintergrund ist dabei nicht unbedingt maßstäblich, und bei manchen Ausführungsbeispielen kann der Anteil des Hintergrundes deutlich größer sein im Verhältnis zum Anteil der Buchstaben. Die Größe der Buchstaben kann zudem auch im Gegensatz zu dem Beispiel der Figuren 17A-17B von Zeile zu Zeile variieren. Die zu untersuchende Person kann dann in Abhängigkeit von wechselnden Linsen (beispielsweise den Linsen 140 und 141) angeben, ob die Buchstaben schärfer oder weniger scharf bzw. besser erkennbar oder weniger gut erkennbar sind. Dabei zeigen die Figuren 17A-17D variierende Umfeldleuchtdichten (Lu) und somit variierenden Kontrast zwischen Buchstaben und Hintergrund, womit verschiedene Lichtverhältnisse und verschiedene Arten des Sehens (skotopisch, mesopisch, photopisch) getestet werden können. Dabei wirkt die Umfeldleuchtdichte insbesondere dann, wenn die jeweils dargestellten Bilder einen großen Teil des Gesichtsfeldes (FoV, vom Englischen: Field of View) ausfüllen, als Adapationsleuchtdichten (La), die die Augenadaption bestimmen. Ein derartiges Gesichtsfeld kann z.B. durch eine entsprechende Betrachtungsoptik, z.B. wie vorstehend diskutiert, erreicht werden. Zusätzlich oder alternativ kann eine Helligkeit der dargestellten Buchstaben eingestellt werden, um eine sogenannte Infeldleuchtdichte (Li) einzustellen. So ist der Kontrast zwischen Buchstaben und Hintergrund in der Fig. 17A am größten und in der Fig. 17D am geringsten. Bei anderen Ausführungsbeispielen können zusätzlich oder alternativ zu verschiedenen Kontrasten und Helligkeiten auch verschiedene Farben verwendet werden.

Zu bemerken ist, dass sich das Ergebnis bei der subjektiven Refraktionsbestimmung von dem Ergebnis der objektiven Refraktionsbestimmung unterscheiden kann. Dies liegt daran, dass sich das menschliche Gehirn bis zu einem gewissen Grad an Fehlsichtigkeit gewöhnen kann und diese kompensieren kann. Wenn nun beispielsweise eine Brille verwendet wird, welche eine Fehlsichtigkeit optisch (objektiv) exakt kompensiert, kann das Gehirn dennoch diese Kompensation zunächst weiterführen, was den subjektiven Bildeindruck dann wieder verschlechtert. Bei Ausführungsbeispielen, welche sowohl objektive Refraktionsbestimmung als auch eine subjektive Refraktionsbestimmung ermöglichen, können beispielsweise beide Werte an medizinisches Fachpersonal übermittelt werden, oder es kann beispielsweise für eine Brillen- oder Kontaktlinsenbestimmung ein Kompromiss aus den Ergebnissen der objektiven Refraktionsbestimmung und der subjektiven Refraktionsbestimmung verwendet werden.

Wie bereits erläutert sind die obigen Ausführungsbeispiele nur zur Veranschaulichung gedacht, und sind nicht als einschränkend auszulegen. Varianten und Abwandlungen, welche bezüglich der objektiven Refraktionsbestimmung diskutiert wurden, können ggf. auch bei der subjektiven Refraktionsbestimmung verwendet werden. Beispielsweise kann auch für die subjektive Refraktionsbestimmung eine Mikrolinsenfolie zur Abbildung bereitgestellt werden. Auch andere Variationen sind möglich.

## Patentansprüche

1. System, umfassend:
eine am Kopf tragbare Halterung,
eine in der Halterung bereitgestellte mobile Computervorrichtung (10; 20; 60), umfassend:
eine Anzeige (11; 21; 61),
einen Prozessor (13), und
einen Speicher mit darin gespeichertem Programmcode (16),
wobei der Programmcode (16), wenn er auf dem Prozessor (13) ausgeführt wird, bewirkt, dass der Prozessor (13) die Anzeige (11) ansteuert, um auf der Anzeige (11) ein Bild zur Refraktionsbestimmung eines Auges (610, 611) anzuzeigen,
wobei in der Halterung bereitgestellt sind:
eine Betrachtungsoptik (62, 63, 100, 101) zum Betrachten der mobilen Computervorrichtung mit einem linken Auge (610) und einem rechten Auge (611) einer zu untersuchenden Person, umfassend Optiken (62, 63), um das Bild auf der Anzeige zu dem linken Auge (610) bzw. dem rechten Auge (611) abzubilden, und
eine Wechselhalterung (143) für eine Wechseloptik (140, 141) zur subjektiven Refraktionsbestimmung.

2. System nach Anspruch 1, **dadurch gekennzeichnet, dass** die Wechselhalterung (143) zur Aufnahme von Wechsellinsen (140, 141) eingerichtet ist und/oder dass in der Wechselhalterung (143) Wechsellinsen (140, 141) aufgenommen sind.

3. System nach einem der Ansprüche 1 oder 2, weiter umfassend, für mindestens ein Auge (610, 611), eine Kombination aus Polarisator (151, 152) und Analysator (153, 154).

4. System nach einem der Ansprüche 1 bis 3, **dadurch gekennzeichnet, dass** die Betrachtungsoptik (62, 63, 100, 101) eine Mikrolinsenanordnung (101) umfasst.

5. System nach Anspruch 4, **dadurch gekennzeichnet, dass** die Mikrolinsenanordnung eine auf der Anzeige (61) der mobilen Computervorrichtung anzubringende Mikrolinsenfolie umfasst.

6. System nach einem der Ansprüche 4-5, **dadurch gekennzeichnet, dass** die Betrachtungsoptik (62, 63, 100, 101) einen Farbfilter (100), einen Rotfilter und/oder einen Infrarotfilter, umfasst.

7. System nach einem der Ansprüche 1-6, weiter umfassend einen Strahlteiler (64, 65), welcher zwischen der Anzeige (61) der mobilen Computervorrichtung (60) und dem mindestens einen Auge (610, 611) angeordnet ist, wobei der Strahlteiler (64, 65) angeordnet ist, von dem Auge (610, 611) ausgehendes Licht zu einer Kameraeinrichtung (66-69) zu lenken.

8. System nach einem der Ansprüche 1-7, weiter umfassend, für mindestens ein Auge, einen Shutter (150).

## Claims

1. System, comprising:
a holder that can be worn on the head,
a mobile computer apparatus (10; 20; 60) provided in the holder, said computer apparatus comprising:
a display (11; 21; 61),
a processor (13), and
a memory with program code (16) stored therein,
wherein the program code (16), when executed on the processor (13), causes the processor (13) to actuate the display (11) in order to display an image for determining the refraction of an eye (610, 611) on the display (11),
wherein the following are provided in the holder:
an observation optical unit (62, 63, 100, 101) for observing the mobile computer apparatus with a left eye (610) and a right eye (611) of a person to be examined, comprising an optical unit (62, 63) for imaging the image on the display onto the left eye (610) and the right eye (611), respectively, and an interchange holder (143) for an interchange optical unit (140, 141) for subjective determination of the refraction.

2. System according to Claim 1, **characterized in that** the interchange holder (143) is configured to receive interchange lens elements (140, 141) and/or in that interchange lens elements (140, 141) are received in the interchange holder (143).

3. System according to either of Claims 1 and 2, further comprising, for at least one eye (610, 611), a combination of polarizer (151, 152) and analyser (153, 154).

4. System according to any one of Claims 1 to 3, **characterized in that** the observation optical unit (62, 63, 100, 101) comprises a microlens arrangement (101).

5. System according to Claim 4, **characterized in that** the microlens arrangement comprises a microlens film to be applied on the display (61) of the mobile computer apparatus.

6. System according to either one of Claims 4 and 5, **characterized in that** the observation optical unit (62, 63, 100, 101) comprises a colour filter (100), a red filter and/or an infrared filter.

7. System according to any one of Claims 1-6, further comprising a beam splitter (64, 65), which is arranged between the display (61) of the mobile computer apparatus (60) and the at least one eye (610, 611), wherein the beam splitter (64, 65) is arranged to steer light emanating from the eye (610, 611) to a camera device (66-69).

8. System according to any one of Claims 1-7, further comprising, for at least one eye, a shutter (150).

## Revendications

1. Système, comprenant :
un support pouvant être porté sur la tête,
un dispositif informatique mobile (10 ; 20 ; 60) prévu dans le support, comprenant :
un afficheur (11 ; 21 ; 61),
un processeur (13), et
une mémoire dans laquelle est stocké un code de programme (16),
dans lequel le code de programme (16), lorsqu'il est exécuté sur le processeur (13), amène le processeur (13) à commander l'affichage (11) afin qu'il affiche sur l'afficheur (11) une image permettant de déterminer la réfraction d'un oeil (610, 611),
dans lequel il est prévu dans le support :
une optique de visualisation (62, 63, 100, 101) permettant de visualiser le dispositif informatique mobile au moyen d'un oeil gauche (610) et d'un oeil droit (611) d'une personne à examiner, comprenant des optiques (62, 63), afin de reproduire l'image sur l'affichage associé à l'oeil gauche (610), respectivement à l'oeil droit (611), et
un support interchangeable (143) destiné à une optique interchangeable (140, 141) pour déterminer la réfraction subjective.

2. Système selon la revendication 1, **caractérisé en ce que** le support interchangeable (143) est destiné à recevoir des lentilles remplaçables (140, 141) et/ou **en ce que** des lentilles remplaçables (140, 141) sont reçues dans le support interchangeable (143).

3. Système selon l'une quelconque des revendications 1 ou 2, comprenant en outre,
pour au moins un oeil (610, 611), une combinaison d'un polariseur (151, 152) et d'un analyseur (153, 154).

4. Système selon l'une quelconque des revendications 1 à 3, **caractérisé en ce que** l'optique de visualisation (62, 63, 100, 101) comprend un ensemble de microlentilles (101).

5. Système selon la revendication 4, **caractérisé en ce que** l'ensemble de microlentilles comprend une feuille de microlentilles destinée à être installée sur l'afficheur (61) du dispositif informatique mobile.

6. Système selon l'une des revendications 4-5, **caractérisé en ce que** l'optique de visualisation (62, 63, 100, 101) comprend un filtre coloré (100), un filtre rouge et/ou un filtre infrarouge.

7. Système selon l'une des revendications 1 à 6, comprenant en outre une séparatrice de faisceau (64, 65) disposée entre l'afficheur (61) du dispositif informatique mobile (60) et l'au moins un oeil (610, 611), dans lequel la séparatrice de faisceau (64, 65) est disposée de manière à diriger la lumière émanant de l'oeil (610, 611) vers un dispositif à caméra (66-69).

8. Système selon l'une des revendications 1-7 comprenant en outre, pour au moins un oeil, un obturateur (150).
